Europäisches Patentamt

European Patent Office

Office européen des brevets

(19)

(11) Publication number: **0 025 967**

**A1**

(12) **EUROPEAN PATENT APPLICATION**

(21) Application number: **80105540.1**

(22) Date of filing: **16.09.80**

(51) Int. Cl.³: **A 61 B 1/00**

(30) Priority: **20.09.79 JP 121224/79**

(43) Date of publication of application:
**01.04.81 Bulletin 81/13**

(84) Designated Contracting States:
**BE CH DE FR GB IT LI NL SE**

(71) Applicant: **OLYMPUS OPTICAL CO., LTD.**
**43-2, 2-chome, Hatagaya Shibuya-ku**
**Tokyo(JP)**

(72) Inventor: **Hattori, Shinichiro**
**2-14, Takamatsu-cho Toshima-ku**
**Tokio(JP)**

(74) Representative: **Kempe, Wolfgang, Dr.**
**Postfach 1273**
**D-6800 Mannheim 1(DE)**

(54) Endoscope system having a tape recorder.

(57) An endoscope system having a tape recorder of the invention comprises an endoscope body 1 of an endoscope A and a light source unit B for supplying light to this endoscope body 1. The body 15 of the tape recorder for inputting sound signals of an operator of the endoscope body 1, a tape recorder operating part 16, and a microphone 14 are assembled in the endoscope A or in the light source unit B. The audio signals of the operator are input when a record button 23 is depressed, or are automatically input by an audio starting device 120.

F I G. 1

EP 0 025 967 A1

0025967

## Background of the Invention

The present invention relates to an endoscope system having a tape recorder.

For performing an examination using an endoscope, a doctor conventionally memorizes his observation results and the parts which were photographed during the examination. He either writes them down on a clinical chart after completing the examination, or writes each time the need arises during the examination. However, with a method which relies on memory, much labor is required for obtaining a large quantity of detailed observations or several tens of photographs. It is not always possible to completely and correctly memorize all of the information. With the method of writing down the information each time when the need arises during an examination, the examination is often interrupted, resulting in complex procedure and inefficiency.

As part of the prior art of the present invention, a laid-open utility model application No. 32805/1980 is known. This utility model application discloses a camera having a tape recorder wherein a case encloses a tape recorder function part and a camera function part for recording the situation.

## Summary of the Invention

It is, therefore, the primary object of the present invention to provide an endoscope system having a tape recorder wherein information such as observation results and parts photographed are recorded as needed with ease even during the operation of the endoscope so that the obtained information may be correct and the doctor may operate the endoscope and examine his patient without interruption.

In order to accomplish the above and other objects, the present invention provides an endoscope having a tape recorder which at least comprises:

an endoscope body with a tape recorder body, an operating part and a microphone assembled therein; and

a light source unit connected to said endoscope body through a cable for providing light to said endoscope body.

Brief Description of the Drawings

Other objects and features of the present invention will be apparent from the following description taken in connection with the accompanying drawings in which:

Fig. 1 is a perspective view illustrating an endoscope system having a tape recorder in accordance with a first embodiment of the present invention;

Fig. 2 is a perspective view illustrating an endoscope system having a tape recorder in accordance with a second embodiment of the present invention;

Fig. 3 is a schematic view of a mechanical part of the tape recorder assembled in the endoscope of the present invention;

Figs. 4A and 4B are electrical control block diagrams of the tape recorder assembled in the endoscope of the present invention;

Figs. 5A through 5G are operating flow charts for controlling the tape recorder according to a permanent program stored in a PROM by an electrical control part shown in Figs. 4A and 4B, wherein Figs. 5A and 5B show the entire block flow chart; Fig. 5C, the block flow chart of a "STOP" sequence subroutine; Fig. 5D, the block flow chart of a "RECORD" sequence subroutine; Fig. 5E, the block flow chart of a "REWIND" sequence subroutine; Fig. 5F, the block flow chart of a "FAST FEED" sequence subroutine; and Fig. 5G, the block flow chart of a "PLAY" sequence subroutine, respectively;

Fig. 6 is a perspective view of an endoscope system having a tape recorder in accordance with a third embodiment of the present invention;

Fig. 7 is a perspective view of an endoscope system having a tape recorder in accordance with a fourth embodiment of the present invention;

Fig. 8 is a block diagram of an audio starting device which may be mounted to the tape recorder of the endoscope system of the present invention; and

Fig. 9 is a detailed circuit diagram of the audio starting device shown in Fig. 8.

Detailed Description of the Preferred Embodiments

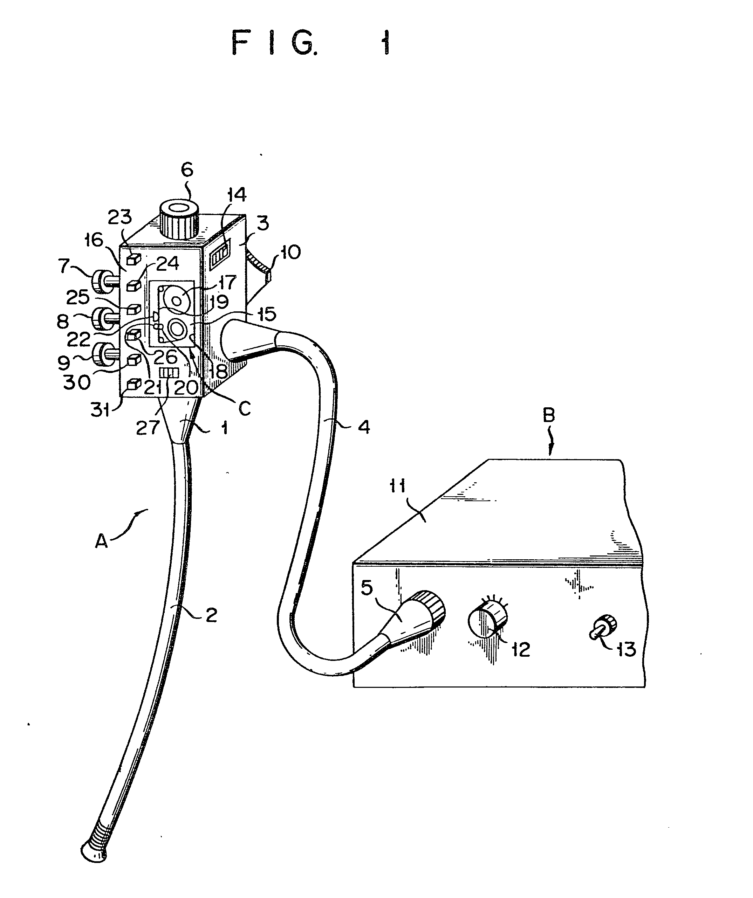

Fig. 1 is a perspective view illustrating the first embodiment of the present invention. The endoscope system comprises an endoscope A and a light source unit B. An endoscope body 1 of the endoscope A comprises an insertion part 2 and an endoscope operating part 3 to which is attached a flexible cable 4. A connector 5 is mounted to the front end of the flexible cable 4 for detachably connecting it to the light source unit B. This endoscope A includes various systems typical of a general endoscope, such as a lighting system, an observation system, air supply means, water supply means, suction means, and so on. The lighting system guides the illuminating light for illuminating the body cavity from the light source unit B to the front end of the insertion part 2 through a light guide (not shown) disposed inside the flexible cable 4, the endoscope operating part 3, and the insertion part 2. The observation system is disposed in the insertion part 2 and the endoscope operating part 3 for enabling observation of the field of the body cavity from an ocular part 6 of the endoscope operating part 3. At the operating part 3 of the endoscope body 1 are disposed operation buttons 7, 8, and 9 for operating the air supply means, the water supply means and the suction means. An angle handle 10 is also included at the endoscope operating part 3 for changing the direction of the front end of the insertion part 2.

A dial 12 for controlling the quantity of light for examination and a toggle switch 13 for the power source switch are mounted on a body 11 of the light source unit B.

A tape recorder C is assembled in the endoscope device of this construction for recording and reproducing the voice of the operator during the operation of the endoscope A. The tape recorder C comprises a microphone 14, a body part 15, and a tape recorder operating part 16, which are all assembled in the endoscope operating part 3 of the endoscope body 1 in the embodiment shown in Fig. 1. The microphone 14 is securely mounted on such a part of the surface that it is near the mouth of the operator when the operator of the endoscope A looks through the ocular part 6. The body part 15 has an encasing chamber 18 for mounting a recording cassette 17, and capstans 20 and pinch rollers 21 for guiding and driving recording tape 19 of the recording cassette 17 for recording and reproducing with a magnetic head 22, a recording circuit, a reproducing circuit and so on. The recording and reproducing circuits may be of conventional type and are well known to those skilled in the art, so they are not shown in the drawings. Particular examples of such circuits are, for example, shown in the U.S. patent application No. 138,195 based on the same application. The recording tape 19 may be driven in the reverse direction for rewinding and may be fast forwarded in the forward direction. The tape recorder operating part 16 includes a record button 23, a rewind button 24, a fast feed button 25, a record inhibit button 26, a play button 30, and a stop button 31 for operating the tape recorder C. The record inhibit button has been incorporated for preventing erroneous recording when the operator erroneously depresses the record button if the record inhibit button has been depressed previously. A tape counter 27 is disposed for displaying the distance that the tape has travelled. In this embodiment, the microphone 14 is also used as a speaker for reproduction. Although a tape recorder having various functions of recording, reproducing, rewinding, fast forwarding, stopping, and record inhibiting is incorporated in the endoscope operating part 3, of this embodiment, a tape recorder which has all these functions except the reproducing function may alternatively be incorporated. Thus, the record button 23, the rewind button 24, the fast feed button 25 and the record inhibit button 26 alone may be disposed in the tape recorder operating part 16 for performing the functions of recording, rewinding, fast forwarding and record inhibition. Reproduction may be performed with another tape recorder having a reproduction function.

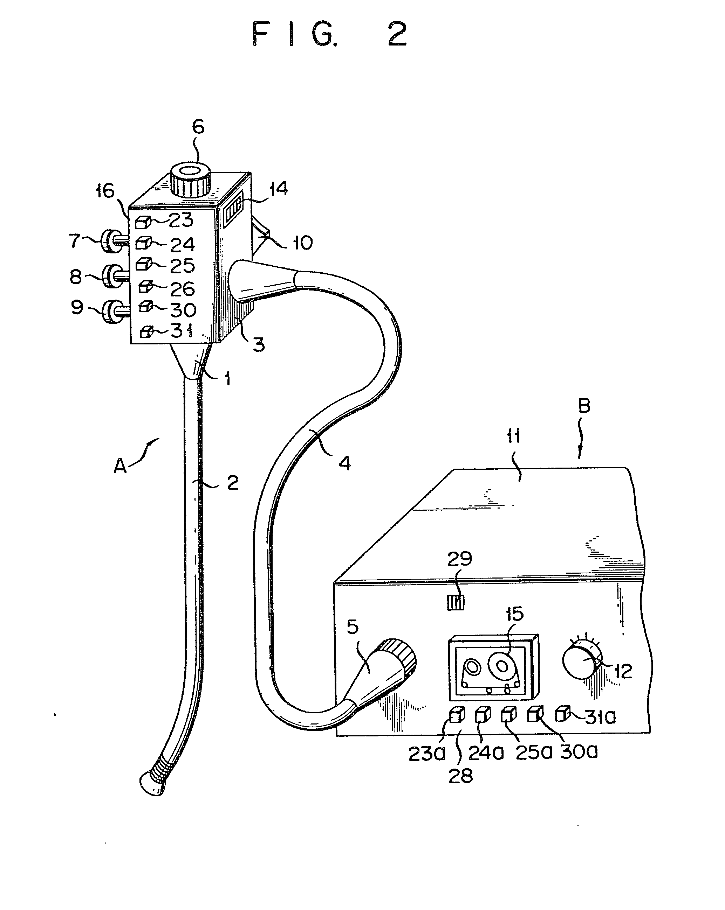

Fig. 2 shows the second embodiment of the present invention wherein the same parts are designated by the same numerals; description thereof is omitted. In this embodiment, the body of the tape recorder C is assembled with the light source unit B, and the microphone 14 and the tape recorder operating part 16 are assembled in the endoscope operating part 3 of the endoscope body. A second tape recorder operating part 28 and a second microphone 29 are mounted in the body 11 of the light source unit B. In this second tape recorder operating part 28, as in the case of the tape recorder operating part 16 at the side of the endoscope operating part 3, are mounted a record button 23a, a rewind button 24a, a fast feed button 25a, a play button 30a, and a stop button 31a so that recording, rewinding, fast forwarding, reproducing, and stopping may also be performed at the light source unit B side. In such a case, the second microphone 29 preferably also functions as a reproducing speaker for saving space. It is also possible to dispose a speaker in the light source unit B separately of the second microphone. Moreover, an earphone jack (not shown) may be formed in the endoscope operating part 3 for feeding the reproducing signal to the endoscope operating part 3 through the connector 5 and the flexible cable 4, so that the operator may be able to hear the reproduced signals by plugging the earphone into the earphone jack without being heard by the patient. Thus, the patient is not psychologically disturbed. Further, a reproducing speaker may be disposed in the endoscope operating part 3, or the microphone 14 may function as a reproducing speaker as well.

With the above-mentioned construction, the operator of the endoscope A may operate the tape recorder operating part 16 of the endoscope operating part 3 for remote control of the tape recorder C. On the other hand, an assistant operates the second tape recorder operating part 28 of the light source unit B so that he can record the number of the clinical chart, the name of the patient and so on separately of the operator of the endoscope A.

In this embodiment, the second microphone 29 at the light source unit B side need not be secured to the body 11 of the light source unit B, but may alternatively be extended through a cord (not shown) for keeping the second microphone 29 at hand while utilizing a microphone jack (not shown).

Fig. 3 schematically shows the mechanical part of the tape recorder shown in Figs. 1 and 2.

When a brake solenoid 41 is turned on, brakes 42 are separated from brake drums 43, 50 so that a supply reel motor 44 and a take-up reel motor 45 are rotatable. When a pinch roller solenoid 46 is turned on next, the supply reel motor 44 is driven weakly in the reverse direction, and the take-up reel motor 45 is driven weakly in the forward direction. As a result, a cassette tape (not shown) is made to travel at a constant speed by a capstan 47 and is wound on the take-up reel inside the cassette. The supply reel motor 44 is driven weakly in the reverse direction for the purpose of providing a suitable tension on the travelling tape. The take-up reel motor 45 is constructed so as to be driven slightly faster than the travelling speed of the tape for winding the tape while providing a suitable tension thereto. A guide pin 48 is disposed for setting the cassette at a predetermined position and fits in a hole formed in the cassette. Reference numerals 49, 50, 51, 52 and 53 denote a capstan motor, a brake drum, a flywheel, a pinch roller and a recording/reproducing head, respectively.

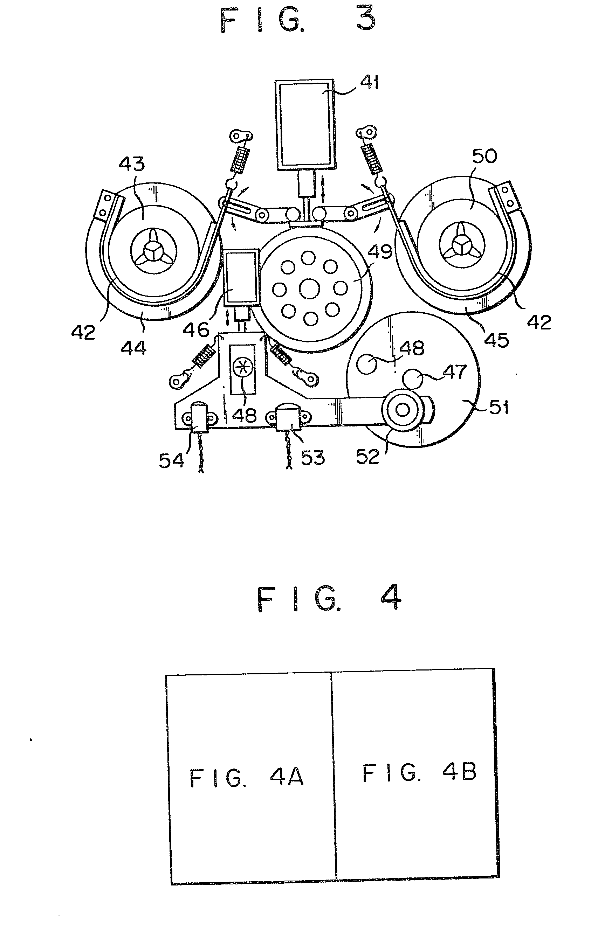

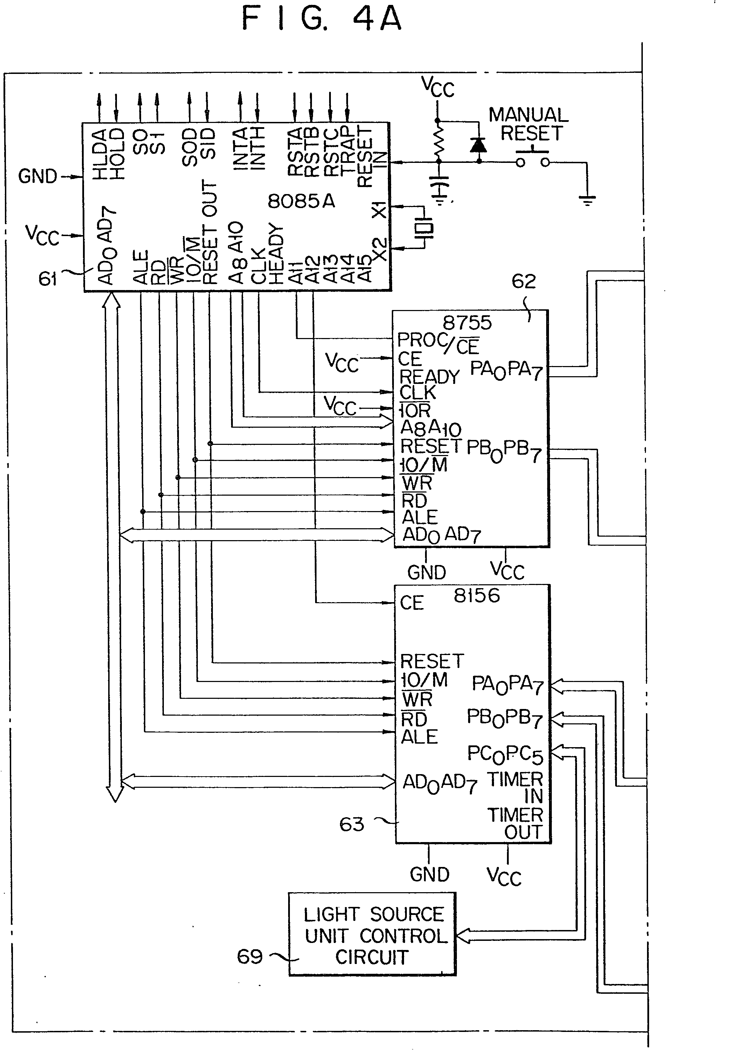

Figs. 4A and 4B show the control part of the tape recorder. The tape recorder has a microcomputer 61. For this microcomputer 61, an 8 bit microcomputer 8085A of Intel Corporation, U.S.A., for example, is suitable. The microcomputer 61 is connected to a first I/O port 62. To this first I/O port 62 is connected a take-up reel motor drive circuit 64, a supply reel motor drive circuit 65, a brake solenoid drive circuit 67, and a pinch roller solenoid drive circuit 68. The take-up reel motor drive circuit 64 is connected to the take-up reel motor 45; the supply reel motor drive circuit 65, to the supply reel motor 44; the brake solenoid drive circuit 67, to the brake solenoid 41; the pinch roller solenoid drive circuit 68, to the pinch roller solenoid 46; and the capstan motor 49, to a capstan motor power source 66, respectively. The first I/O port 62 has a PROM (programmable read-only memory). In this PROM part are stored programs for controlling the take-up reel motor drive circuit 64, the supply reel motor drive circuit 65, a capstan motor drive circuit 66, the brake solenoid drive circuit 67, and the pinch roller solenoid drive circuit 68 for each operation of recording, rewinding, fast forwarding, record inhibiting, reproducing and stopping. Also stored in the PROM is a program for controlling a light source unit control circuit 69 to be described hereinafter. Although a PROM capable of rewriting is used in this embodiment, an ROM which is not capable of rewriting once it has been written for example a fuse ROM, can also be used. The microcomputer 61 is connected to a second I/O port 63. The second I/O port 63 is connected to the record button 23, the rewind button 24, the fast feed button 25, the record inhibit button 26, the play button 30, and the stop button 31, which are all disposed on the endoscope body A. The microcomputer 61 is also connected to the record button 23a, the rewind button 24a, the fast feed button 25a, the play button 30a, and the stop button 31a, which are disposed at the light source unit B side in the second embodiment in a manner to be described later.

The second I/O port 63 is connected to a light source unit control circuit 69. This light source unit control circuit 69 controls the lighting conditions of the examination, and its construction and operation are described in a U.S. patent application No. 122,175 entitled "Strobe Photography Device" of Takayama and in a U.S. patent application No. 133,668 entitled "Automatic Exposure Device for Endoscope" of Takayama of the same applicant. The RAM part is used for an operating area for various logic and arithmetic operations and as a memory area for the input data.

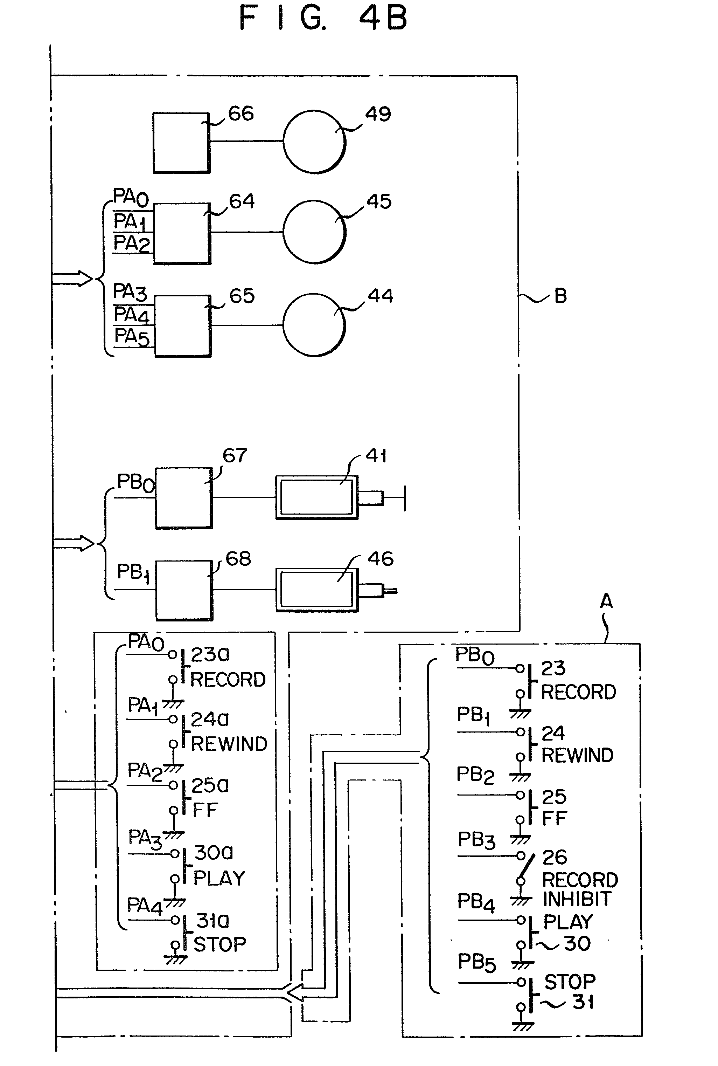

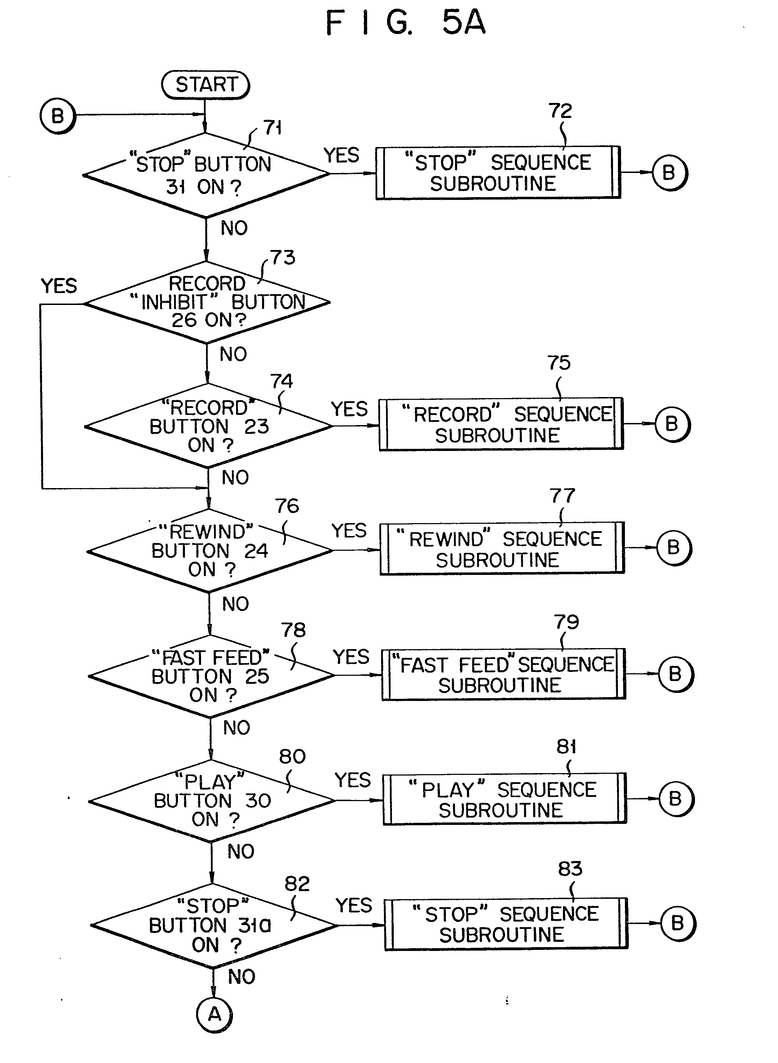

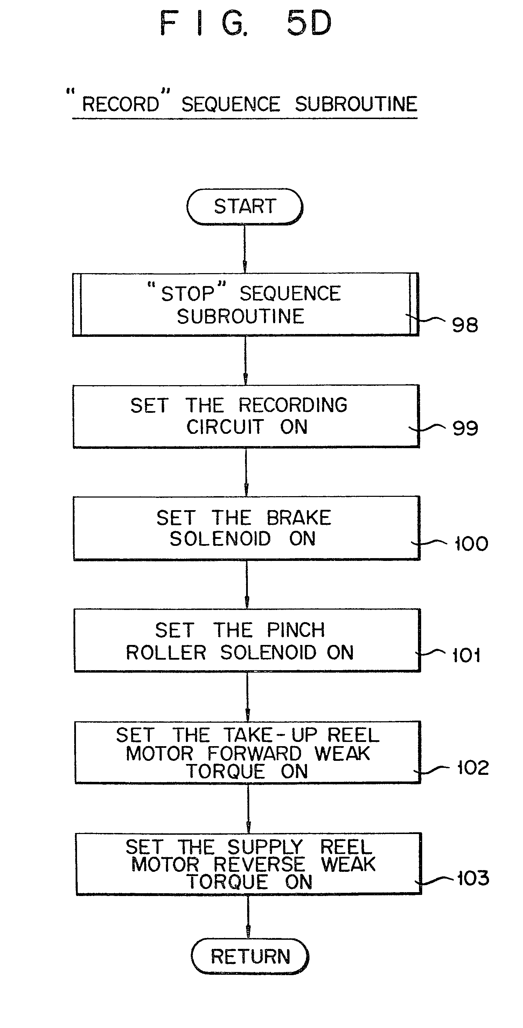

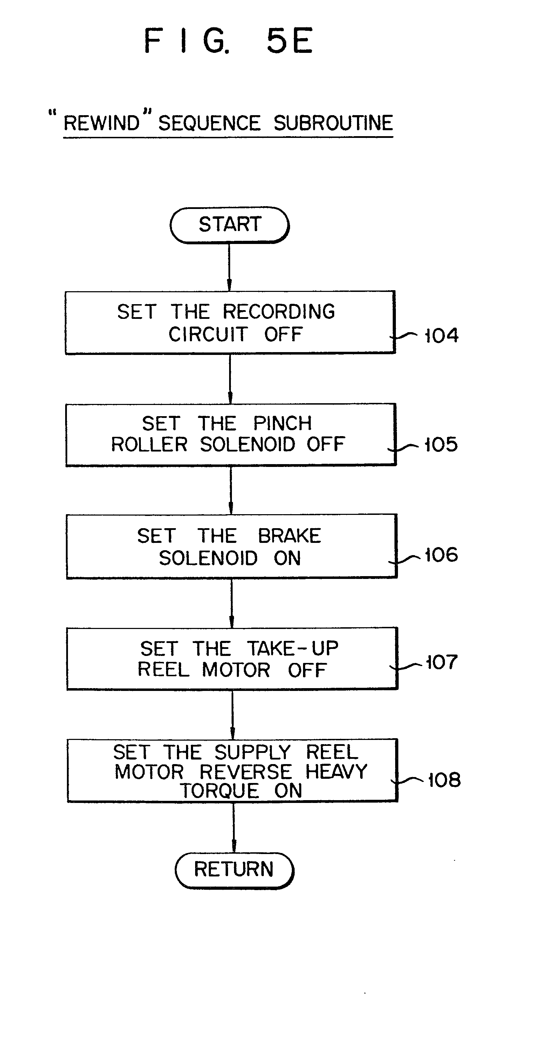

Each of the operations controlled by the permanent program stored in the PROM part of the first I/O port 62 when the record button 23, the rewind button 24, the fast feed button 25, the record inhibit button 26, the play button 30, and the stop button 31 in the embodiment shown in Fig. 1 are depressed will be described referring to Fig. 5A.

When the stop button 31 is depressed in a step 71, the stop sequence subroutine is called. (See step 72). When the record inhibit button 26 has been depressed previously, the control does not proceed to step 74, a step 76 follows, and when the record button 23 is depressed in a step 74, a step 75 follows for calling the record sequence subroutine. When the rewind button 24 is depressed in the step 76, a step 77 follows for calling the rewind sequence subroutine. When the fast feed button 25 is depressed in a step 78, a step 79 follows for calling the fast feed sequence subroutine. When the play button 30 is depressed in a step 80, a step 81 follows for calling the play sequence subroutine. In the case of the first embodiment, the operation returns back to the point B of Fig. 5A and the above steps are repeated.

The above-mentioned stop sequence subroutine, the record sequence subroutine, the rewind sequence subroutine, the fast feed sequence subroutine, and the play sequence subroutine will be described in detail later.

Each of the operation flows controlled by the semi-permanent program stored in the PROM part of the first I/O port 62 when the record button 23, the rewind button 24, the fast feed button 25, the record inhibit button 26, the play button 30, and the stop button 31 in the second embodiment shown in Fig. 2 will be described referring to Figs. 5A through 5G. When the stop button 31 is depressed in the step 71 of Fig. 5A, the stop sequence subroutine is called (See step 72). When the record inhibit button 26 has been depressed previously, the control does not proceed to step 74 and the record operation is inhibited, the step 76 follows, and when the record button 23 is depressed in the step 74, the step 75 follows for calling the record sequence subroutine. When the rewind button 24 is depressed in the step 76, the step 77 follows for calling the rewind sequence subroutine. When the fast feed button 25 is depressed in the step 78, the step 79 follows for calling the fast feed sequence subroutine. When the play button 30 is depressed in the step 80, the step 81 follows for calling the play sequence subroutine. This is the control flow effected when the buttons of the operation part 16 of the tape recorder at the side of the endoscope body 1 are operated. In the case of the second embodiment, the first tape recorder operating part is disposed at the side of the endoscope body 1 and the second tape recorder operating part is disposed at the side of the light source unit, so that when they are simultaneously operated confirmation is made as to whether or not any operating button of the tape recorder operating part 16 at the side of the endoscope body 1 has been depressed, and thereafter the tape recorder operating part at the side of the light source unit B is checked for giving priority to the tape recorder operating part 16 at the side of the endoscope body 1.

A step 82 is then initiated for checking whether any of the buttons 23a, 24a, 25a, 26a, 30a, and 31a of the tape recorder operating part at the side of the light source unit B has been depressed. When the stop button 31a is depressed in the step 82, a step 83 follows and the stop sequence subroutine is called.

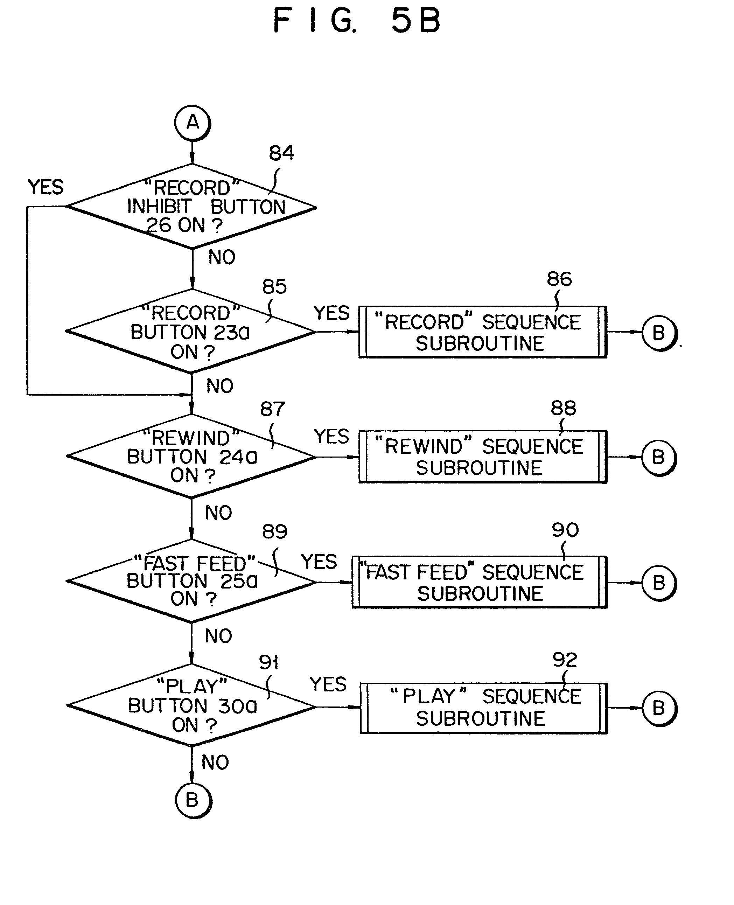

Then a step 84 as shown in Fig. 5B follows. When the record inhibit button 26 at the side of the endoscope body 1 has been depressed previously, a step 87 follows. When the record button 23a is depressed in a step 85, the record sequence subroutine is called in a step 86. When the rewind button 24a is depressed in the step 87, the rewind sequence subroutine is called in a step 88. When the fast feed button 25a is depressed in a step 89, the fast feed sequence subroutine is called in a step 90. When the play button 30a is depressed in a step 91, the play sequence subroutine is called in a step 92.

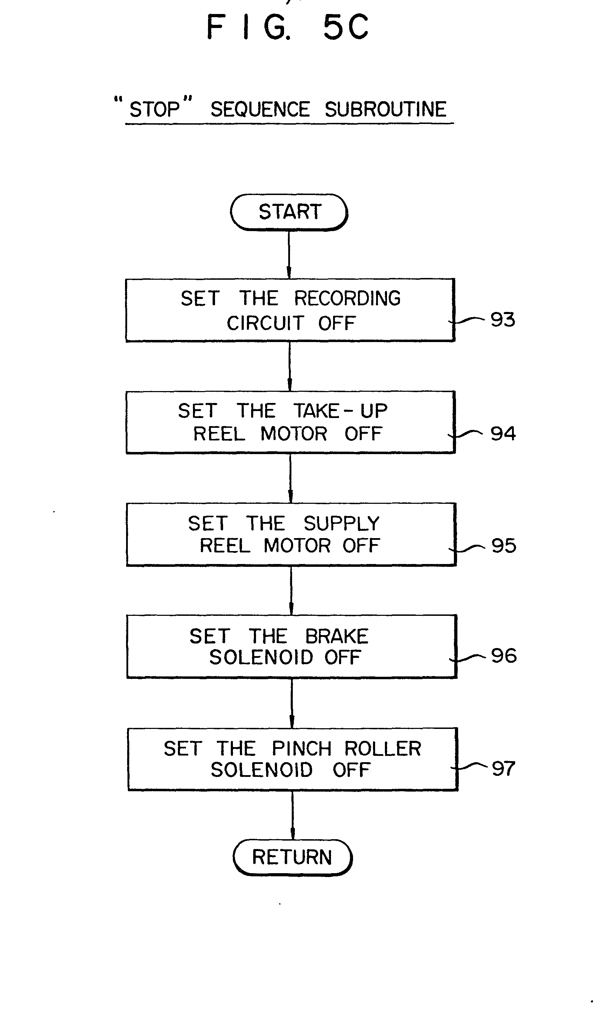

Each sequence subroutine will now be described with reference to Figs. 5C through 5G.

The stop sequence subroutine is to deactivate the recording circuit (not shown) in a step 93. For attaining this, the recording/reproducing head 53 is separated from the recording circuit to connect with the play circuit so that the supply of erasing current to the erasing head is interrupted. Then the take-up reel motor 45 is deactivated in a step 94 so that a step 95 is initiated. In the step 95, the supply reel motor 44 is deactivated. The brake solenoid 41 is deactivated in a step 96, and the pinch roller solenoid 46 is deactivated in a step 97.

In the record sequence subroutine, the stop sequence subroutine is called in a step 98. The recording circuit is then activated in a step 99. The brake solenoid 41 is activated, and the pinch roller solenoid 46 is activated (Steps 100, 101). The take-up reel motor 45 assumes a weak forward torque in a step 102, and the supply reel motor 44 assumes a weak reverse torque in a step 103.

In the rewind sequence subroutine as shown in Fig. 5E, the recording circuit is deactivated in a step 104 and the pinch roller solenoid is deactivated in a step 105. In a step 106, the brake solenoid 41 is activated, and the take-up reel motor 45 is deactivated in a step 107. In a step 108, the supply reel motor 44 assumes a heavy reverse torque.

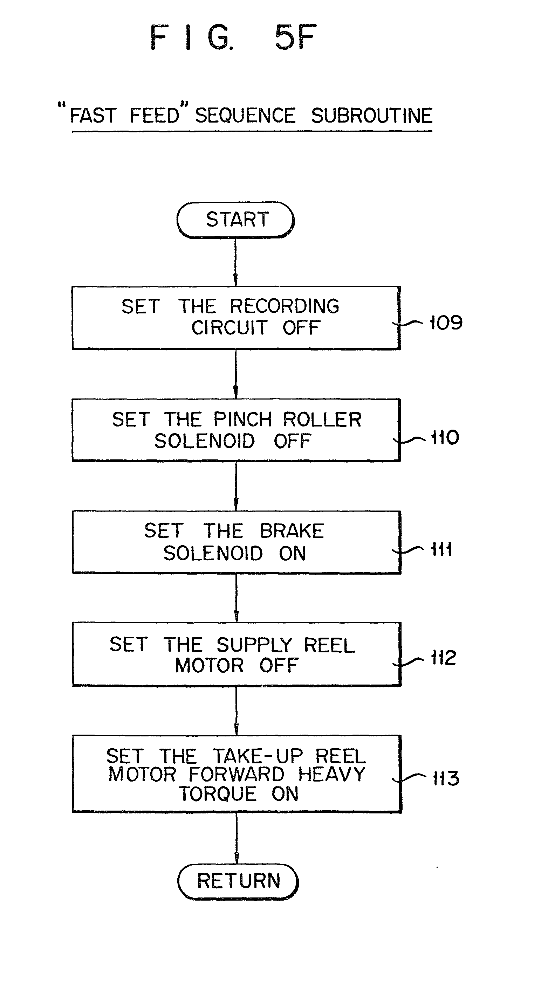

Fig. 5F shows the fast feed sequence subroutine. In a step 109, the recording circuit is deactivated. The pinch roller solenoid 46 is deactivated in a step 110. In a step 111, the brake solenoid 41 is deactivated so that a step 112 follows. In the step 112, the supply reel motor 44 is deactivated, and in a step 113 the take-up reel motor 45 assumes a heavy forward torque.

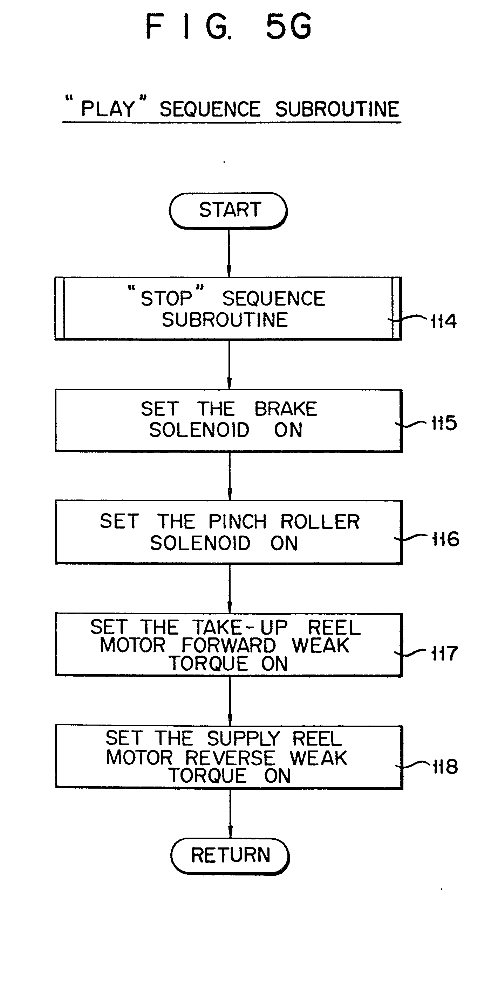

The play sequence subroutine is shown in Fig. 5G, wherein the stop sequence subroutine is first called in a step 114. In a step 115, the brake solenoid 41 is activated, and the pinch roller solenoid 46 is activated in a step 116 so that a step 117 follows. The take-up reel motor 45 assumes a weak forward torque in the step 117, and the supply reel motor 44 assumes a weak reverse torque in a step 118.

The method for using the endoscope of the first embodiment will now be described.

The recording cassette 17 is mounted in the encasing chamber 18. If immediate recording is not desired, the record inhibit button 26 is depressed so as to prevent erroneous recording which may be caused when the record button 23 is depressed by accident.

When the operator of the endoscope A has obtained his observation results and desires to record by photography, he releases the record inhibit button 26 and speaks in the microphone 14 while depressing the record button 23. The tape recorder C can be operated and used for recording only while the record button 23 is being depressed. Thus, the operator is able to record his observation results and required information about parts of the photographs taken and so on even while the endoscope A is operating.

The rewind button 24 is depressed for rewinding the recording tape 19. The fast feed button 25 is depressed for fast forwarding.

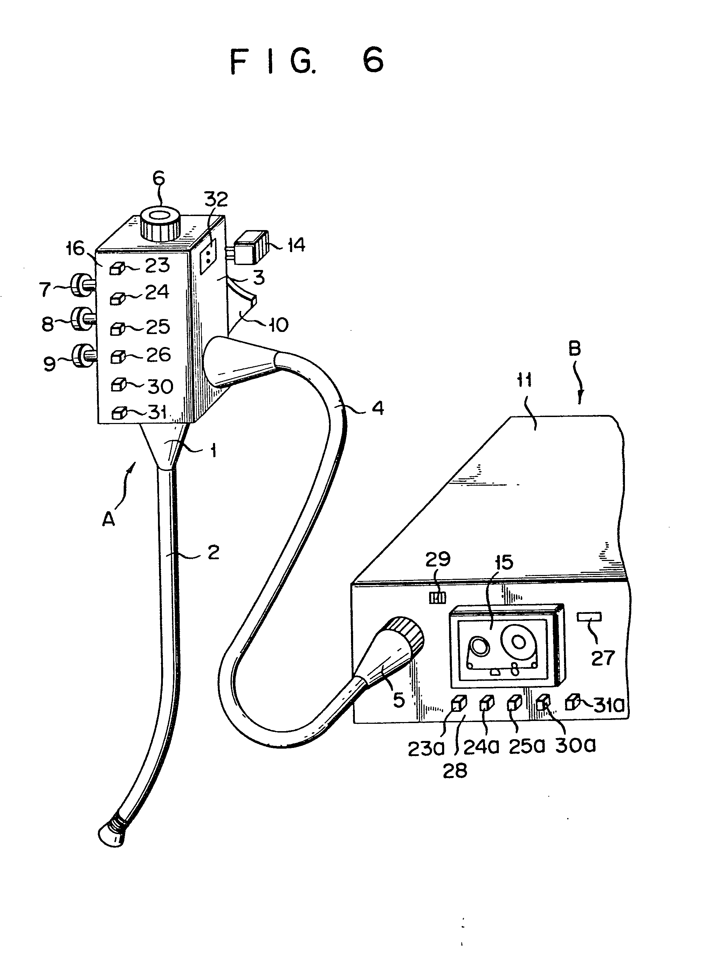

Fig. 6 shows the third embodiment of the present invention. In the figure, the same parts are designated by the same reference numerals as in Fig. 1, and description of these parts will be omitted. In this embodiment, the microphone 14 at the endoscope A side is detachably mounted. A water-tight microphone socket 32 is mounted at the endoscope operating part 3, and the microphone 14 is detachably mounted thereto.

Although the microphone 14 is damaged by liquids, the microphone can be detached while the endoscope A is cleaned and sterilized, so that cleansing solutions and chemicals may be freely used for the endoscope A. Moreover, since the microphone can be detached, the endoscope can be made highly resistant to high temperatures and high pressure so that the endoscope may be sterilized in an autoclave if necessary.

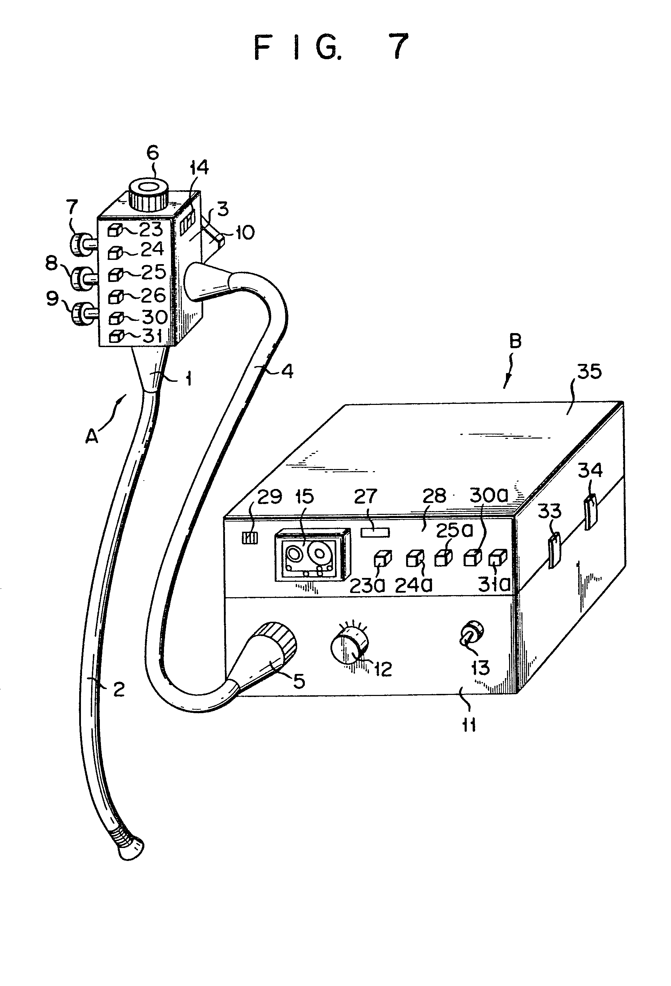

Fig. 7 shows the fourth embodiment of the present invention wherein the same parts are designated by the same reference numerals, and the description of these parts will be omitted. In this embodiment, a box body 35 is disposed separately from the body 11 of the light source unit B in which are assembled the body part 15 of the tape recorder C, the second tape recorder operating part 28 and the second microphone 29.

This box body 35 is connected to the body 11 by metal fasteners 33, 34 and is so constructed as to be separable therefrom by a one-touch mechanism.

In the above embodiment, recording is effected by depressing the record button. However, it is also possible to include an audio starting device 120 in the tape recorder C for automatically driving the tape recorder by an audio input signal fed to the microphone. With this audio starting device for the tape recorder, the switching circuit connected to the power source operates in response to an audio signal fed to the recording microphone of the tape recorder for connecting the power source to an electrical circuit of the tape recorder, so that the tape recorder may be automatically placed in recording mode.

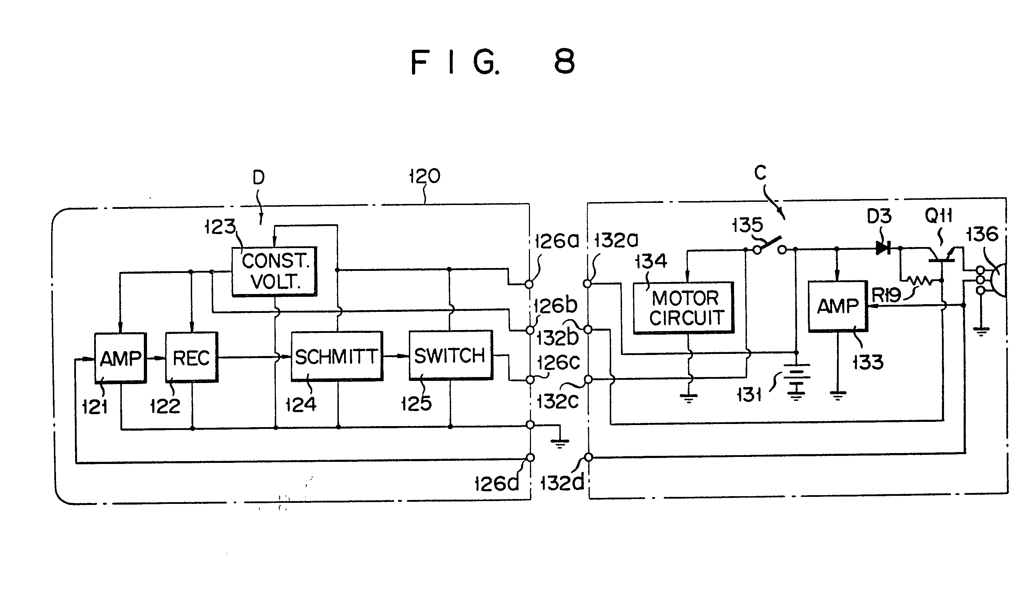

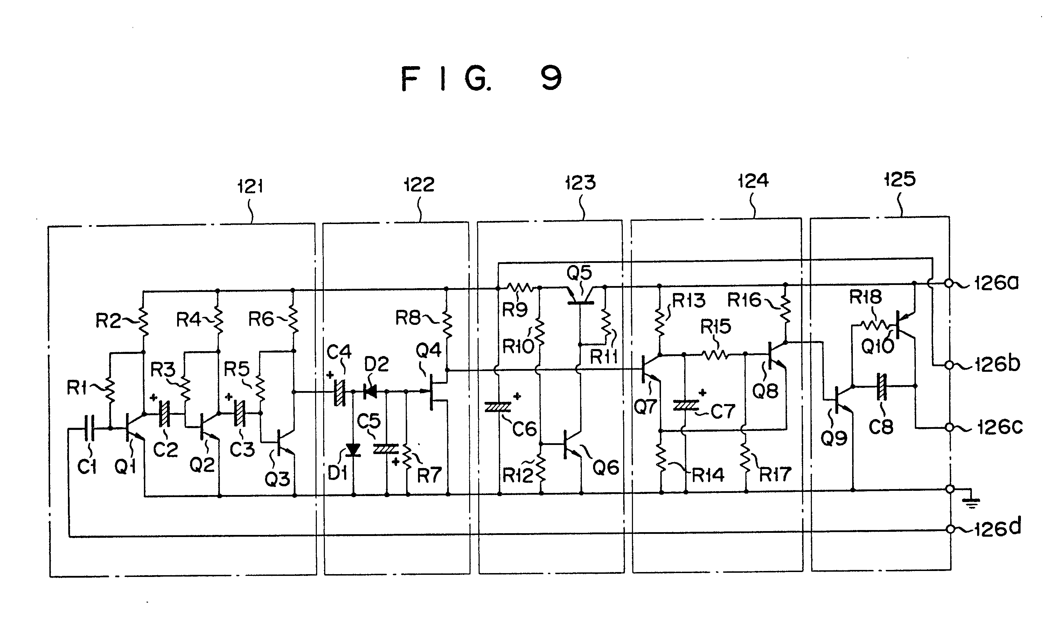

The audio starting device 120, as shown in Fig. 8, comprises an amplifying circuit 121, a rectifying circuit 122, a constant voltage circuit 123, a Schmitt circuit 124, and a transistor switching circuit 125. The amplifying circuit 121 is a three-stage amplifying type which comprises transistors Q1, Q2, and Q3; capacitors C1, C2, and C3; and resistors R1 through R6 as shown in Fig. 9. The amplifying circuit 121 thus amplifies the output of the microphone which is input through a connecting terminal 126d for feeding it to the rectifying circuit 122 of the next stage. The rectifying circuit 122 comprises an FET transistor Q4; capacitors C4 and C5; resistors R7, R8; and diodes D1 and D2. The rectifying circuit 122 rectifies the output of the amplifying circuit 121 and feeds it to the Schmitt circuit 124.

The constant voltage circuit 123 comprises transistors Q5, Q6; a capacitor C6; and resistors R9 through R12. When the power source of the tape recorder C is connected to the audio starting circuit through a connecting terminal 126a, the constant voltage circuit 123 stabilizes the operating voltage from this power source and applies the stabilized voltage to each of the circuits 121, 122, 123, 124, and 125 through a decoupling circuit of the resistor R0 and the capacitor C6.

The Schmitt circuit 124 comprises transistors Q7, Q8; a capacitor C7; and resistors R13 - R17, and it determines the switching level of the transistor switching circuit 125. The Schmitt circuit 124 operates in response to the output of the rectifying circuit 122.

The transistor switching circuit 125 comprises transistors Q9, Q10; a capacitor C8; and a resistor R18 for performing switching operations by the output of the Schmitt circuit 124. When this circuit 125 performs a switching operation, the transistor Q10 is rendered conductive so that the operating voltage is supplied to the electrical circuit of the tape recorder C through a connecting terminal 126c connected to the collector of the transistor Q10. When the transistor Q10 is deactivated from the activated condition, the electrical circuit consisting of the resistor R18 and the capacitor C8 operates so that the transistor Q10 is gradually deactivated. The recovery time of the audio starting circuit is determined by the capacitor C5 and the resistor R7.

A connecting terminal 126b is connected to the junction of the capacitor C6 and the resistor R9 of the decoupling circuit. When the audio starting device D is coupled to the tape recorder C, as shown in Fig. 8, the operating voltage renders a switching transistor Q11 conductive for applying the operating voltage to the terminal 126b and the built-in microphone of the tape recorder C.

In the tape recorder C, a built-in power source 131 supplies the operating voltage to a connecting terminal 132a and an amplifying circuit 133. A motor circuit 134 has a pause switch 135 which is opened when the operation of the tape recorder C is automatically controlled by the audio starting device D. For audio starting, the operating voltage of the motor circuit 134 is applied through the transistor switching circuit 125 in the starting device D and connecting terminals 126c, 132c. The voltage of the power source 131 is applied to the operating circuit of a built-in microphone 136 through a diode D3 for preventing reverse flow of current. The operating circuit comprises the switching transistor Q11 and a resistor R19. The power source voltage is applied to the collector of the transistor Q11 through the diode D3. The transistor Q11 is rendered conductive when a specified voltage is applied to the base, and the transistor Q11 feeds the operating voltage to the built-in microphone 136. For the built-in microphone 136, an electret capacitor microphone of high performance is used, and its output terminal is connected to the amplifying circuit and a connecting terminal 132d.

The operating voltage of the built-in microphone 136 is supplied from the microphone power source supply terminal (not shown) in the tape recorder C during the normal period, that is, at times other than the audio starting period. Since the connecting terminals 126a through 126d of the audio starting device D are in contact with the connecting terminals 132a through 132d of the tape recorder C, respectively, the electrical circuit of the audio starting device D is connected to the electrical circuit of the tape recorder. Under this condition, the pause switch 135 of the tape recorder C is open, and the tape recorder is operated in the recording mode.

When these are thus coupled, the connecting terminal 126a contacts the connecting terminal 132a so that the built-in power source 131 of the tape recorder C is connected to the electrical circuit of the audio starting device D and are placed under prepared condition.

Since the connecting terminal 126d contacts the connecting terminal 132d, the output end of the built-in microphone 136 of the tape recorder C is connected to the input end of the amplifying circuit of the audio starting circuit D. Since the connecting terminal 126c contacts the connecting terminal 132c, the output end of the transistor switching circuit 125 of the audio starting circuit D is connected to the operating voltage supply terminal of the motor circuit 134 of the tape recorder C.

Since the connecting terminal 126b contacts the connecting terminal 132b, the output voltage of the constant voltage circuit 123 is applied to the base of the switching transistor Q11 for the microphone operating circuit of the tape recorder C. This transistor is rendered conductive when this voltage is applied, so that the voltage of the power source 131 is applied to the built-in microphone 136 through the diode D3 and the transistor Q11. The microphone is thus operated, and the operation of the tape recorder C is completely controlled by the audio starting device D.

When a sound is input to the built-in microphone 136 under this condition, the output of the microphone is fed as an input to the amplifying circuit of the audio starting circuit, so that the audio starting circuit operates and the transistor Q10 of the switching circuit 125 conducts. When this is rendered conductive, the voltage of the power source 131 is applied to the motor circuit 134 of the tape recorder C in the circuit of the connecting terminals 126a, 132a to the transistor Q10 to the connecting terminals 126c, 132c, so that the motor circuit 134 operates and the motor is activated to drive the tape. Since this series of operations is enacted in a very short period of time, the motor is driven almost simultaneously as the sound is supplied to the microphone 136. Thus, there is no operation lag of the motor, so that the recording operation of the tape recorder is automatically controlled by the audio starting device.

With the audio starting device, the operator of the endoscope does not need to depress the record button 23 every time the need to record arises, so that he can restrict himself entirely to performing the operation of the endoscope A.

0025967

- 1 -

Claims:

1. An endoscope system having an endoscope body 1 and a light source unit B connected to said endoscope body 1 through a cable for supplying light to said endoscope body 1 characterized in that

said endoscope body 1 has a body 15, an operating part 16, and a microphone 14 of a tape recorder C for inputting and recording the voice of an operator of said endoscope system when said endoscope system is operated by the operator.

2. An endoscope system as recited in claim 1 characterized in that

said operating part 16 comprises at least

a record button 23 for inputting an audio signal;

a rewind button 24 for rewinding a tape of said tape recorder C;

a fast feed button 25 for fast forwarding the tape of said tape recorder C; and

a stop button 31 for interrupting the travel of the tape of said tape recorder C.

3. An endoscope system as recited in claim 1 characterized in that

said endoscope body 1 further has a tape counter 27 for displaying the distance the tape has travelled.

4. An endoscope system having an endoscope body 1 and a light source unit B connected to said endoscope body 1 through a cable for supplying light to said endoscope body 1 characterized in that

said endoscope body 1 has an operating part 16 and a microphone 14 of a tape recorder for inputting and recording the voice of an operator when said endoscope body 1 is operated by the operator by remote control; and

said light source unit B has a tape recorder body 15 and a second tape recorder operating part 28 for inputting to at least record and reproduce the voice of an operator of said endoscope body 1 when directly operated

0025967

by an operator of said light source unit B.

5. An endoscope system as recited in claim 4 characterized in that

said light source unit B further has a tape counter 27 for displaying the distance the tape has travelled.

6. An endoscope system having an endoscope body 1 and a light source unit B connected to said endoscope body 1 through a cable for supplying light to said endoscope body 1 characterized in that

said endoscope body 1 has an operating part 16 and a microphone 14 of a tape recorder for inputting and recording the voice of an operator when said endoscope system is operated by the operator by remote control, and said light source unit B has a recording unit 35 which is detachable from said light source unit B and which has a tape recorder body 15 and a second tape recorder operating part of a tape recorder for at least recording and reproducing the voice of an operator of said endoscope body 1 when directly operated by an operator of said light source unit B.

7. An endoscope as recited in claim 6 characterized in that

said recording unit 35 has a tape counter 27 for displaying the distance the tape has travelled.

8. An endoscope system as recited in claim 4 or 6 characterized in that

said first operating part 16 comprises

a record button 23 for inputting an audio signal;

a rewind button 24 for rewinding the tape of said tape recorder C;

a fast feed button 25 for fast forwarding the tape of said tape recorder C;

a stop button 31 for interrupting the travel of the tape of said tape recorder C; and

a play button 30 for reproducing information recorded on the tape; and

said second operating part 28 comprises

a record button 23a for inputting an audio signal;

a rewind button 24a for rewinding the tape of said tape recorder C;

a fast feed button 25a for fast forwarding the tape of said tape recorder C;

a stop button 31a for interrupting the travel of the tape of said tape recorder C; and

a play button 30a for reproducing information recorded on the tape.

9. An endoscope system as recited in claim 1, 4, or 6 characterized in that a microphone 14 is detachably mounted in said endoscope body.

10. An endoscope system as recited in claim 4 characterized in that

said light source unit B further has a second microphone 29 for inputting the voice of an operator of said light source unit B.

11. An endoscope system as recited in claim 6 characterized in that

said recording unit further has a second microphone 29 for inputting the voice of an operator of said recording unit.

12. An endoscope system as recited in claim 1, 4, or 6 characterized in that

said body 15 of said tape recorder C has

a central processing element 61 connected to at least a take-up reel motor drive circuit 64, a brake solenoid drive circuit 67 and a pinch roller solenoid drive circuit 68 through an I/O port 62 for supplying control signals to said drive circuits 64, 67, 68;

a read-only memory 62 for storing a permanent program, connected to said central processing element 61 and said drive circuits 64, 67, 68 for controlling said drive circuits; and

a random access memory 63 which is connected to said record buttons 23, 23a, said rewind buttons 24, 24a, said fast feed buttons 25, 25a, said play buttons 30, 30a, said stop buttons 31, 31a and said light source unit control circuit 69 for temporarily storing the information input from said record buttons 23, 23a, said rewind buttons 24, 24a, said fast feed buttons 25, 25a, said play buttons 30, 30a and said stop buttons 31, 31a and which is also connected to said light source control circuit 69 for temporarily storing the control information between said light source unit control circuit 69 and said central processing circuit 61.

1/16

F I G. 1

0025967

# F I G. 2

F I G. 3

F I G. 4

FIG. 4A   FIG. 4B

# F I G. 4A

5/16

# F I G. 4B

0025967

F I G. 5A

START

B →

"STOP" BUTTON 31 ON ? — 71 — YES → "STOP" SEQUENCE SUBROUTINE — 72 → B

NO

RECORD "INHIBIT" BUTTON 26 ON ? — 73 — YES →

NO

"RECORD" BUTTON 23 ON ? — 74 — YES → "RECORD" SEQUENCE SUBROUTINE — 75 → B

NO

"REWIND" BUTTON 24 ON ? — 76 — YES → "REWIND" SEQUENCE SUBROUTINE — 77 → B

NO

"FAST FEED" BUTTON 25 ON ? — 78 — YES → "FAST FEED" SEQUENCE SUBROUTINE — 79 → B

NO

"PLAY" BUTTON 30 ON ? — 80 — YES → "PLAY" SEQUENCE SUBROUTINE — 81 → B

NO

"STOP" BUTTON 31a ON ? — 82 — YES → "STOP" SEQUENCE SUBROUTINE — 83 → B

NO

A

7/16

# F I G. 5 B

8/16

# F I G. 5C

"STOP" SEQUENCE SUBROUTINE

```
              ( START )
                  │
                  ▼
        ┌─────────────────────┐
        │   SET  THE  RECORDING│ ──── 93
        │      CIRCUIT  OFF    │
        └─────────────────────┘
                  │
                  ▼
        ┌─────────────────────┐
        │   SET  THE  TAKE-UP  │ ──── 94
        │    REEL  MOTOR  OFF  │
        └─────────────────────┘
                  │
                  ▼
        ┌─────────────────────┐
        │    SET  THE  SUPPLY  │ ──── 95
        │    REEL  MOTOR  OFF  │
        └─────────────────────┘
                  │
                  ▼
        ┌─────────────────────┐
        │    SET  THE  BRAKE   │ ──── 96
        │     SOLENOID  OFF    │
        └─────────────────────┘
                  │
                  ▼
        ┌─────────────────────┐
        │  SET  THE  PINCH ROLLER│ ──── 97
        │     SOLENOID  OFF    │
        └─────────────────────┘
                  │
                  ▼
             ( RETURN )
```

9/16

# F I G.  5D

"RECORD" SEQUENCE SUBROUTINE

```
        ┌─────────┐
        │  START  │
        └─────────┘
             │
             ▼
   ┌──────────────────────┐
   │  "STOP" SEQUENCE      │ ── 98
   │   SUBROUTINE          │
   └──────────────────────┘
             │
             ▼
   ┌──────────────────────┐
   │  SET THE RECORDING    │ ── 99
   │   CIRCUIT ON          │
   └──────────────────────┘
             │
             ▼
   ┌──────────────────────┐
   │  SET THE BRAKE        │ ── 100
   │   SOLENOID ON         │
   └──────────────────────┘
             │
             ▼
   ┌──────────────────────┐
   │  SET THE PINCH        │ ── 101
   │   ROLLER SOLENOID ON  │
   └──────────────────────┘
             │
             ▼
   ┌──────────────────────┐
   │  SET THE TAKE-UP REEL │ ── 102
   │   MOTOR FORWARD WEAK  │
   │    TORQUE ON          │
   └──────────────────────┘
             │
             ▼
   ┌──────────────────────┐
   │  SET THE SUPPLY REEL  │ ── 103
   │   MOTOR REVERSE WEAK  │
   │    TORQUE ON          │
   └──────────────────────┘
             │
             ▼
        ┌─────────┐
        │ RETURN  │
        └─────────┘
```

# F I G. 5E

"REWIND" SEQUENCE SUBROUTINE

```
          ( START )
              │
              ▼
┌──────────────────────────┐
│   SET THE RECORDING       │
│      CIRCUIT  OFF         │───── 104
└──────────────────────────┘
              │
              ▼
┌──────────────────────────┐
│    SET  THE  PINCH        │
│  ROLLER SOLENOID OFF      │───── 105
└──────────────────────────┘
              │
              ▼
┌──────────────────────────┐
│   SET  THE  BRAKE         │
│     SOLENOID  ON          │───── 106
└──────────────────────────┘
              │
              ▼
┌──────────────────────────┐
│   SET  THE  TAKE-UP       │
│   REEL MOTOR  OFF         │───── 107
└──────────────────────────┘
              │
              ▼
┌──────────────────────────┐
│  SET THE SUPPLY  REEL     │
│ MOTOR  REVERSE HEAVY      │───── 108
│      TORQUE  ON           │
└──────────────────────────┘
              │
              ▼
          ( RETURN )
```

11/16

# F I G.  5F

"FAST FEED" SEQUENCE SUBROUTINE

```
                    ┌─────────────┐
                    │    START    │
                    └─────────────┘
                           │
                           ▼
            ┌──────────────────────────┐
            │   SET  THE  RECORDING    │── 109
            │      CIRCUIT  OFF        │
            └──────────────────────────┘
                           │
                           ▼
            ┌──────────────────────────┐
            │   SET  THE  PINCH ROLLER │── 110
            │     SOLENOID  OFF        │
            └──────────────────────────┘
                           │
                           ▼
            ┌──────────────────────────┐
            │    SET  THE  BRAKE       │── 111
            │     SOLENOID  ON         │
            └──────────────────────────┘
                           │
                           ▼
            ┌──────────────────────────┐
            │   SET  THE  SUPPLY REEL  │── 112
            │      MOTOR  OFF          │
            └──────────────────────────┘
                           │
                           ▼
            ┌──────────────────────────┐
            │  SET  THE  TAKE-UP REEL  │
            │  MOTOR FORWARD HEAVY     │── 113
            │      TORQUE  ON          │
            └──────────────────────────┘
                           │
                           ▼
                    ┌─────────────┐
                    │   RETURN    │
                    └─────────────┘
```

# F I G. 5G

## "PLAY" SEQUENCE SUBROUTINE

```
        ┌─────────────┐
        │    START    │
        └─────────────┘
               │
               ▼
   ╔═══════════════════════╗
   ║   "STOP"  SEQUENCE    ║──── 114
   ║     SUBROUTINE        ║
   ╚═══════════════════════╝
               │
               ▼
   ┌───────────────────────┐
   │   SET  THE  BRAKE     │──── 115
   │    SOLENOID  ON       │
   └───────────────────────┘
               │
               ▼
   ┌───────────────────────┐
   │  SET  THE PINCH ROLLER│──── 116
   │     SOLENOID  ON      │
   └───────────────────────┘
               │
               ▼
   ┌───────────────────────┐
   │  SET  THE TAKE-UP REEL│──── 117
   │  MOTOR FORWARD WEAK   │
   │      TORQUE  ON       │
   └───────────────────────┘
               │
               ▼
   ┌───────────────────────┐
   │  SET  THE SUPPLY REEL │──── 118
   │  MOTOR REVERSE WEAK   │
   │      TORQUE  ON       │
   └───────────────────────┘
               │
               ▼
        ┌─────────────┐
        │   RETURN    │
        └─────────────┘
```

# F I G. 6

F I G. 7

# F I G. 8

FIG. 9

# EUROPEAN SEARCH REPORT

**European Patent Office**

0025967

Application number

EP 80105540.1

| | DOCUMENTS CONSIDERED TO BE RELEVANT | | | CLASSIFICATION OF THE APPLICATION (Int. Cl. ³) |
|---|---|---|---|---|
| Category | Citation of document with indication, where appropriate, of relevant passages | Relevant to claim | | |
| A | DE - A1 - 2 741 714 (OLYMPUS OPT.) <br> + Figure 4; pages 13-16 + <br> -- | 1 | | A 61 B 1/00 |
| A | DE - A1 - 2 657 852 (OLYMPUS OPT.) <br> + Figure 1; page 5 + <br> -- | 1 | | |
| A | CH - A - 555 581 (H. KRUSE) <br> + Totality + <br> ---- | | | |

**TECHNICAL FIELDS SEARCHED (Int.Cl. ³)**

A 61 B 1/00
G 11 B 5/00
G 11 B 15/00
G 11 B 31/00
A 61 B 19/00
G 06 F 15/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| X | The present search report has been drawn up for all claims | | |
|---|---|---|---|
| Place of search <br> VIENNA | Date of completion of the search <br> 16-12-1980 | Examiner <br> LUDWIG |

EPO Form 1503.1 06.78